# EUROPEAN PATENT SPECIFICATION

(11) **EP 4 213 743 B1**
(45) Date of publication and mention of the grant of the patent: **23.07.2025**
(21) Application number: 22786477.4
(22) Date of filing: 23.09.2022
(51) Int. Cl.: A61B 17/072, A61B 17/115

(54) **OVERLAPPING STAPLE PATTERN FOR SURGICAL STAPLER**
ÜBERLAPPENDES KLAMMERMUSTER FÜR CHIRURGISCHES KLAMMERGERÄT
MOTIF D'AGRAFES SE CHEVAUCHANT POUR AGRAFEUSE CHIRURGICALE

(30) Priority: 27.09.2021 US 202117485589
(43) Date of publication of application: 26.07.2023
(73) Proprietor: Cilag GmbH International, 6300 Zug (CH)
(72) Inventor: SCHINGS, Brian D., Cincinnati, Ohio 45242 (US); MAYHAUS, Francesca N., Cincinnati, Ohio 45242 (US)
(74) Representative: Carpmaels & Ransford LLP
(86) International application number: PCT/IB2022/059020
(87) International publication number: WO 2023/047351

(56) References cited:
- EP-A2- 0 072 754
- EP-A2- 3 000 407
- WO-A1-96/22055
- US-A- 5 571 285
- US-A1- 2014 263 570
- US-A1- 2018 206 844

## Description

### BACKGROUND

Examples of surgical instruments include surgical staplers, which may be configured for use in laparoscopic surgical procedures and/or open surgical procedures. Some such staplers are operable to clamp down on layers of tissue, cut through the clamped layers of tissue, and drive staples through the layers of tissue to substantially seal the severed layers of tissue together near the severed ends of the tissue layers. Examples of surgical staplers are disclosed in U.S. Pat. No. 7,404,508, entitled "Surgical Stapling and Cutting Device," issued July 29, 2008; U.S. Pat. No. 7,434,715, entitled "Surgical Stapling Instrument Having Multistroke Firing with Opening Lockout," issued October 14, 2008; U.S. Pat. No. 7,721,930, entitled "Disposable Cartridge with Adhesive for Use with a Stapling Device," issued May 25, 2010; U.S. Pat. No. 8,408,439, entitled "Surgical Stapling Instrument with An Articulatable End Effector," issued April 2, 2013; and U.S. Pat. No. 8,453,914, entitled "Motor-Driven Surgical Cutting Instrument with Electric Actuator Directional Control Assembly," issued June 4, 2013.

While various kinds of surgical stapling instruments and associated components have been made and used, it is believed that no one prior to the inventor(s) has made or used the invention described in the appended claims.

US 5571285 A discloses a method whereby staple legs in alternate rows in the surgical stapler are made to interlock.

### SUMMARY

The present invention is defined in the appended claims. Embodiments are set out in the dependent claims.

### BRIEF DESCRIPTION OF THE DRAWINGS

The accompanying drawings, which are incorporated in and constitute a part of this specification, illustrate embodiments of the invention, and, together with the general description of the invention given above, and the detailed description of the embodiments given below, serve to explain the principles of the present invention.
FIG. 1 depicts a perspective view of an example of an articulating surgical stapling instrument;
FIG. 2 depicts a side view of the instrument of FIG. 1;
FIG. 3 depicts a perspective view of an opened end effector of the instrument of FIG. 1;
FIG. 4A depicts a side cross-sectional view of the end effector of FIG. 3, taken along line 4-4 of FIG. 3, with a firing beam in a proximal position;
FIG. 4B depicts a side cross-sectional view of the end effector of FIG. 3, taken along line 4-4 of FIG. 3, with the firing beam in a distal position;
FIG. 5 depicts an end cross-sectional view of the end effector of FIG. 3, taken along line 5-5 of FIG. 3;
FIG. 6 depicts an exploded perspective view of the end effector of FIG. 3;
FIG. 7 depicts a perspective view of the end effector of FIG. 3, positioned at tissue and having been actuated once in the tissue;
FIG. 8A depicts a schematic perspective view of first exemplary alternative staples in a non-deformed state, which may be incorporated into the instrument of FIG. 1;
FIG. 8B depicts a schematic perspective view of the staples of FIG. 8A in a deformed state where portions of the staples overlap one another;
FIG. 9A depicts a schematic top view of the staples of FIG. 8A;
FIG. 9B depicts a schematic top view of the staples of FIG. 8B;
FIG. 10 depicts a schematic side elevational view of the staples of FIG. 8B;
FIG. 11 depicts a schematic end view of the staples of FIG. 8B;
FIG. 12A depicts an end view of second exemplary alternative staples in a non-deformed state spaced from a first exemplary alternative anvil, shown in cross-section, which may be incorporated into the instrument of FIG. 1;
FIG. 12B depicts an end view of the staples of FIG. 12A in a deformed state against the anvil of FIG. 12A where portions of the staples overlap one another;
FIG. 13A depicts a side elevational view of the staples and the anvil of FIG. 12A;
FIG. 13B depicts a side elevational view of the staples and the anvil of FIG. 12B;
FIG. 14A depicts a bottom view of the staples and the anvil of FIG. 12A;
FIG. 14B depicts a bottom view of the staples and the anvil of FIG. 12B;
FIG. 15A depicts a top view of the staples of FIG. 12A;
FIG. 15B depicts a top view of the staples of FIG. 15A, but in a partially deformed state;
FIG. 15C depicts a top view of the staples of FIG. 15B, but in the deformed state of FIG. 12B;
FIG. 16 depicts a perspective view of the staples of FIG. 12B;
FIG. 17 depicts a perspective view of an arrangement of staples including the staples of FIG. 12B;
FIG. 18 depicts a top view of the arrangement of staples of FIG. 17;
FIG. 19 depicts a perspective view of non-overlapping staples in a deformed state that are spaced apart from one another;
FIG. 20 depicts a top view of the plurality of staples of FIG. 19;
FIG. 21 depicts a perspective view of a second exemplary alternative anvil that may be incorporated into the instrument of FIG. 1;
FIG. 22 depicts a top view of the anvil of FIG. 21;
FIG. 23 depicts a perspective view of a third exemplary alternative anvil that may be incorporated into the instrument of FIG. 1;
FIG. 24 depicts a top view of the anvil of FIG. 23;
FIG. 25 depicts a perspective view of an exemplary circular surgical stapler, with a battery pack removed from a handle assembly and an anvil assembly removed from a stapling head assembly;
FIG. 26 depicts a top view of a fourth exemplary alternative anvil for use with anvil assembly of FIG. 25;
FIG. 26A depicts cross-sectional view of the anvil of FIG. 26 taken along line 26A-26A of FIG. 26; and
FIG. 27 depicts a top view of third exemplary alternative staples in a deformed state, where the staples may be incorporated into a circular surgical stapler.

The drawings are not intended to be limiting in any way, and it is contemplated that various embodiments of the invention may be carried out in a variety of other ways, including those not necessarily depicted in the drawings. The accompanying drawings incorporated in and forming a part of the specification illustrate several aspects of the present invention, and together with the description serve to explain the principles of the invention; it being understood, however, that this invention is not limited to the precise arrangements shown.

### DETAILED DESCRIPTION

The following description of certain examples of the invention should not be used to limit the scope of the present invention. Other examples, features, aspects, embodiments, and advantages of the invention will become apparent to those skilled in the art from the following description, which is by way of illustration, one of the best modes contemplated for carrying out the invention. As will be realized, the invention is capable of other different and obvious aspects, all without departing from the invention. Accordingly, the drawings and descriptions should be regarded as illustrative in nature and not restrictive.

### I. Exemplary Surgical Stapler

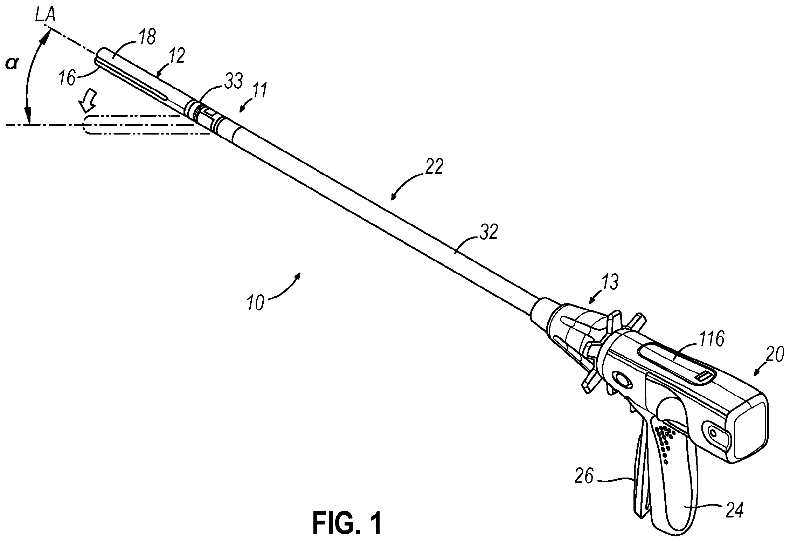

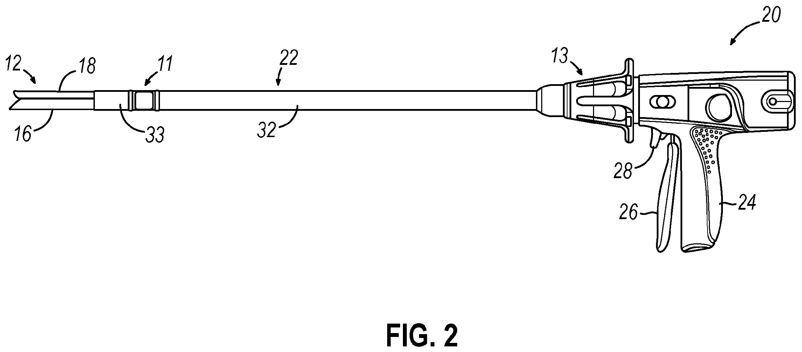

FIGS. 1-7 depict an example of a surgical stapling and severing instrument (10) that is sized for insertion, in a nonarticulated state as depicted in FIG. 1, through a trocar cannula, thoracotomy, or other incision to a surgical site in a patient for performing a surgical procedure. Instrument (10) of the present example includes a handle portion (20) connected to a shaft (22). Shaft (22) distally terminates in an articulation joint (11), which is further coupled with an end effector (12). It should be understood that terms such as "proximal" and "distal" are used herein with reference to a clinician gripping handle portion (20) of instrument (10). Thus, end effector (12) is distal with respect to the more proximal handle portion (20).

Once articulation joint (11) and end effector (12) are inserted into the patient, articulation joint (11) may be remotely articulated, as depicted in phantom in FIG. 1, by an articulation control (13), such that end effector (12) may be deflected from the longitudinal axis (LA) of shaft (22) at a desired angle (α). By way of example only, articulation joint (11) and/or articulation control (13) may be constructed and operable in accordance with at least some of the teachings of U.S. Pat. No. 9,186,142, entitled "Surgical Instrument End Effector Articulation Drive with Pinion and Opposing Racks," issued on November 17, 2015; and/or U.S. Pat. No. 9,795,379, entitled "Surgical Instrument with Multi-Diameter Shaft," issued October 24, 2017. Other suitable forms that articulation joint (11) and articulation control (13) may take will be apparent to those skilled in the art in view of the teachings herein.

End effector (12) of the present example includes a lower jaw (16) and an upper jaw in the form of a pivotable anvil (18). By way of example only, lower jaw (16) may be constructed and operable in accordance with at least some of the teachings of U.S. Pat. No. 9,808,248, entitled "Installation Features for Surgical Instrument End Effector Cartridge," issued November 7, 2017. Anvil (18) may be constructed and operable in accordance with at least some of the teachings of at least some of the teachings of U.S. Pat. No. 10,092,292, entitled "Staple Forming Features for Surgical Stapling Instrument," issued October 9, 2018. Other suitable forms that lower jaw (16) and anvil (18) may take will be apparent to those skilled in the art in view of the teachings herein.

Handle portion (20) includes a pistol grip (24) and a closure trigger (26). Closure trigger (26) is pivotable toward pistol grip (24) to cause clamping, or closing, of the anvil (18) toward lower jaw (16) of end effector (12). Such closing of anvil (18) is provided through a closure tube (32) and a closure ring (33), which both longitudinally translate relative to handle portion (20) in response to pivoting of closure trigger (26) relative to pistol grip (24). Closure tube (32) extends along the length of shaft (22); and closure ring (33) is positioned distal to articulation joint (11). Articulation joint (11) is operable to transmit longitudinal movement from closure tube (32) to closure ring (33).

Handle portion (20) also includes a firing trigger (28). An elongate member (not shown) longitudinally extends through shaft (22) and communicates a longitudinal firing motion from handle portion (20) to a firing beam (14) in response to actuation of firing trigger (28). This distal translation of firing beam (14) causes the stapling and severing of tissue clamped in end effector (12), as will be described in greater detail below. Thereafter, triggers (26, 28) may be released to release the tissue from end effector (12).

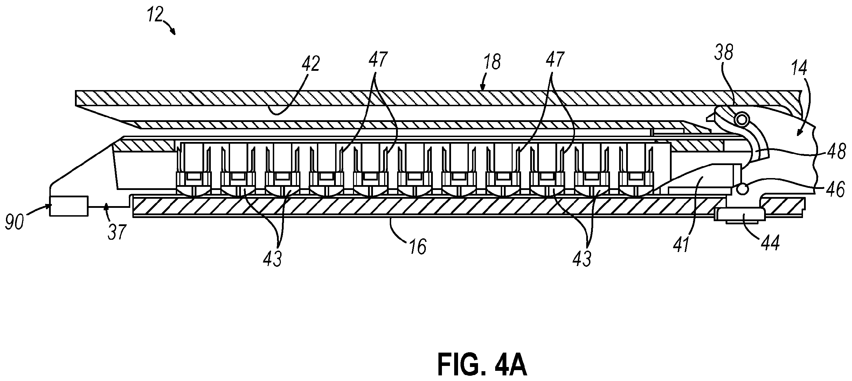

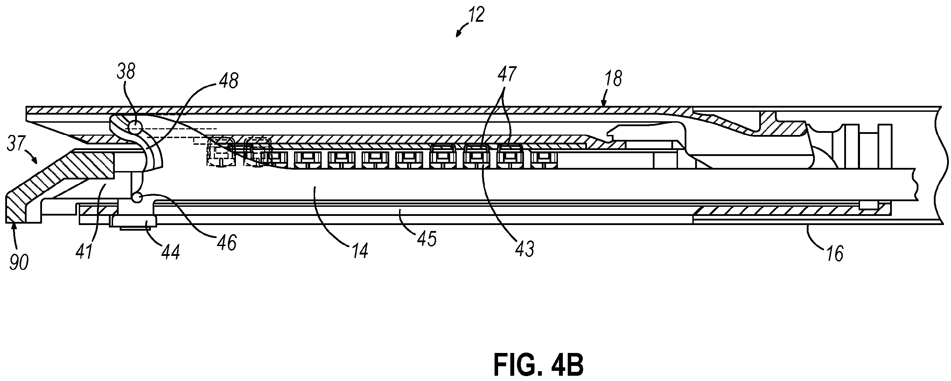

As best seen in FIGS. 4A-4B, firing beam (14) of the present example includes a transversely oriented upper pin (38), a firing beam cap (44), a transversely oriented middle pin (46), and a distally presented cutting edge (48). Upper pin (38) is positioned and translatable within a longitudinal anvil slot (42) of anvil (18). Firing beam cap (44) slidably engages a lower surface of lower jaw (16) by having firing beam (14) extend through lower jaw slot (45) (shown in FIG. 4B) that is formed through lower jaw (16). Middle pin (46) slidingly engages a top surface of lower jaw (16), cooperating with firing beam cap (44). Thereby, firing beam (14) affirmatively spaces end effector (12) during firing. By way of example only, firing beam (14) and/or associated lockout features may be constructed and operable in accordance with at least some of the teachings of U.S. Pat. No. 9,717,497, entitled "Lockout Feature for Movable Cutting Member of Surgical Instrument," issued August 1, 2017. Other suitable forms that firing beam (14) may take will be apparent to those skilled in the art in view of the teachings herein.

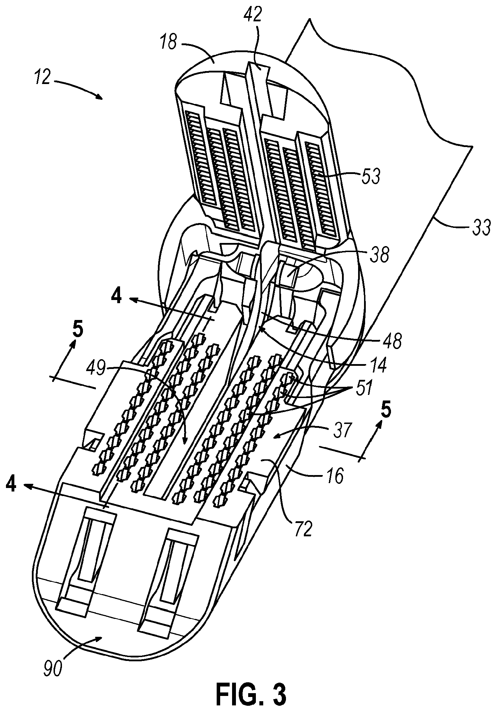

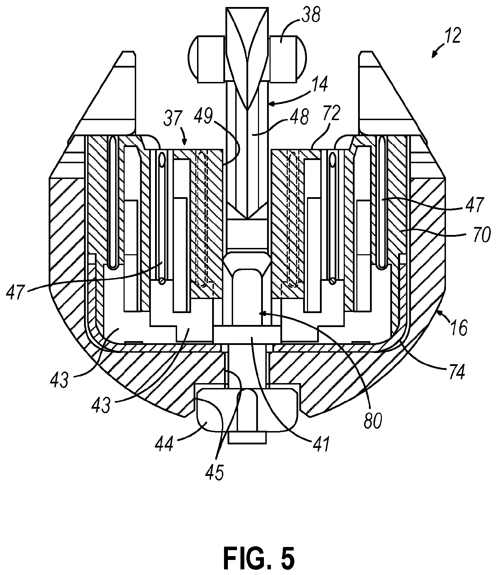

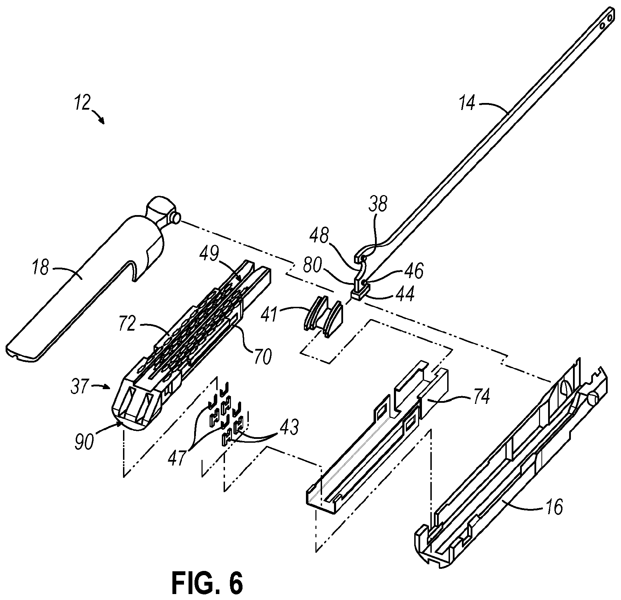

FIG. 3 shows firing beam (14) of the present example proximally positioned and anvil (18) pivoted to an open position, allowing an unspent staple cartridge (37) to be removably installed into a channel of lower jaw (16). As best seen in FIGS. 5-6, staple cartridge (37) of this example includes a cartridge body (70), which presents an upper deck (72) and is coupled with a lower cartridge tray (74). As best seen in FIG. 3, a vertical slot (49) is formed through part of staple cartridge (37). Three rows of staple apertures (51) are formed through upper deck (72) on one side of vertical slot (49), with another set of three rows of staple apertures (51) being formed through upper deck (72) on the other side of vertical slot (49). Of course, any other suitable number of staple rows (e.g., two rows, four rows, any other number) may be provided. Referring back to FIGS. 4A-6, a wedge sled (41) and a plurality of staple drivers (43) are captured between cartridge body (70) and tray (74), with wedge sled (41) being located proximal to staple drivers (43) when staple cartridge (37) is in a pre-fired (or "unspent") state. Wedge sled (41) is movable longitudinally within staple cartridge (37); while staple drivers (43) are movable vertically within staple cartridge (37). Staples (47) are also positioned within cartridge body (70), above corresponding staple drivers (43). In particular, each staple (47) is driven vertically within cartridge body (70) by a staple driver (43) to drive staple (47) out through an associated staple aperture (51). As best seen in FIGS. 4A-4B and 6, wedge sled (41) presents inclined cam surfaces that urge staple drivers (43) upwardly as wedge sled (41) is driven distally through staple cartridge (37).

By way of example only, staple cartridge (37) may be constructed and operable in accordance with at least some of the teachings of U.S. Pat. No. 9,517,065, entitled "Integrated Tissue Positioning and Jaw Alignment Features for Surgical Stapler," issued December 13, 2016. Other suitable forms that staple cartridge (37) may take will be apparent to those skilled in the art in view of the teachings herein.

With end effector (12) closed as depicted in FIGS. 4A-4B by distally advancing closure tube (32) and closure ring (33), firing beam (14) is then advanced in engagement with anvil (18) by having upper pin (38) enter longitudinal anvil slot (42). A pusher block (80) (shown in FIG. 5) located at the distal end of firing beam (14) is configured to engage wedge sled (41) such that wedge sled (41) is pushed distally by pusher block (80) as firing beam (14) is advanced distally through staple cartridge (37) when firing trigger (28) is actuated. During such firing, cutting edge (48) of firing beam (14) enters vertical slot (49) of staple cartridge (37), severing tissue clamped between staple cartridge (37) and anvil (18). As shown in FIGS. 4A-4B, middle pin (46) and pusher block (80) together actuate staple cartridge (37) by entering into vertical slot (49) within staple cartridge (37), driving wedge sled (41) into upward camming contact with staple drivers (43) that in turn drive staples (47) out through staple apertures (51) and into forming contact with staple forming pockets (53) (shown in FIG. 3) on the inner surface of anvil (18). FIG. 4B depicts firing beam (14) fully distally translated after completing severing and stapling of tissue. It should be understood that staple forming pockets (53) are intentionally omitted from the view in FIGS. 4A-4B; but staple forming pockets (53) are shown in FIG. 3. It should also be understood that anvil (18) is intentionally omitted from the view in FIG. 5.

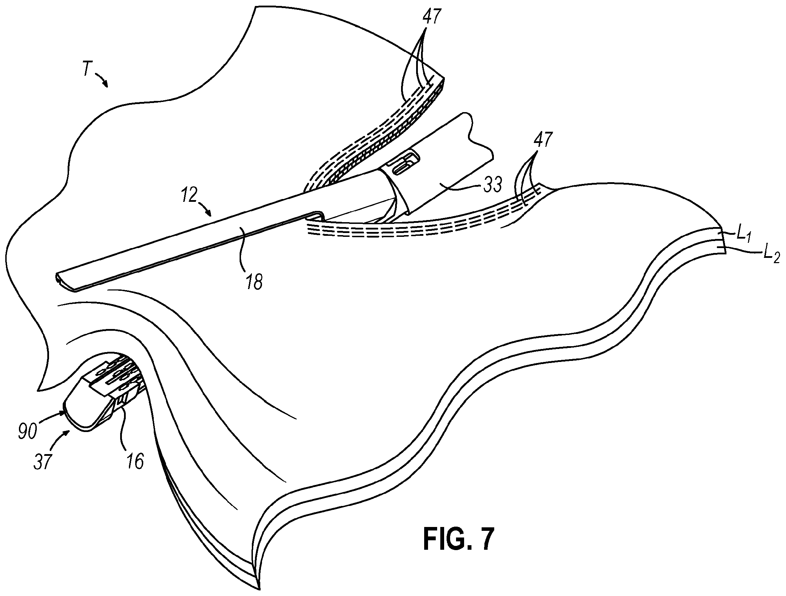

FIG. 7 shows end effector (12) having been actuated through a single stroke through layers (L₁, L₂) of tissue (T). As shown, cutting edge (48) (obscured in FIG. 7) has cut through tissue (T), while staple drivers (43) have driven three alternating rows of staples (47) through the tissue (T) on each side of the cut line produced by cutting edge (48). Staples (47) are all oriented substantially parallel to the cut line in this example, though it should be understood that staples (47) may be positioned at any suitable orientations. In the present example, end effector (12) is withdrawn from the trocar or incision after the first stroke is complete, spent staple cartridge (37) is replaced with a new staple cartridge, and end effector (12) is then again inserted through the trocar or incision to reach the stapling site for further cutting and stapling. This process may be repeated until the desired number of cuts and staples (47) have been provided. Anvil (18) may need to be closed to facilitate insertion and withdrawal through the trocar; and anvil (18) may need to be opened to facilitate replacement of staple cartridge (37).

In some versions, instrument (10) provides motorized control of firing beam (14). By way of example only, such motorization may be provided in accordance with at least some of the teachings of U.S. Pat. No. 9,622,746, entitled "Distal Tip Features for End Effector of Surgical Instrument," issued April 18, 2017; and/or U.S. Pat. No. 8,210,411, entitled "Motor-Driven Surgical Instrument," issued July 3, 2012. Other suitable components, features, and configurations for providing motorization of firing beam (14) will be apparent to those skilled in the art in view of the teachings herein. It should also be understood that some other versions may provide manual driving of firing beam (14), such that a motor may be omitted.

### II. Exemplary Staples and Exemplary Anvils

It may be desirable to modify staples (47) of staple cartridge (37) and/or anvil (18) described above to increase staple density. As used herein, staple density is intended to refer to the number of staples per unit area of the staple cartridge. Higher staple density may provide increased seal strength against luminal leakage through the formed staple pattern. It is also beneficial to reduce time and associated costs of manufacturing, inspecting, and qualifying anvil (18) for use with instrument (10) of FIG. 1. It may also be beneficial to reduce the dimensions of anvil (18) while still providing adequate stapling functionality so that instrument (10) may more easily access the desired anatomy of the patent.

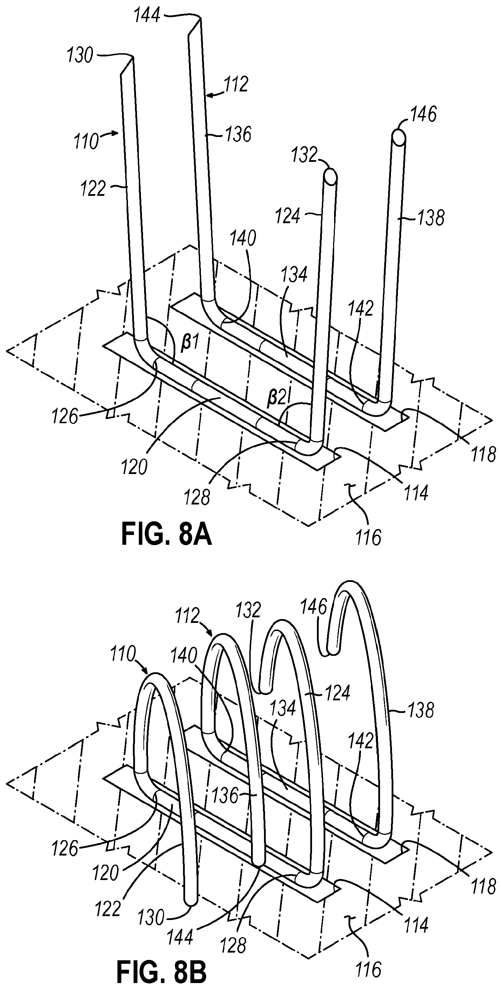

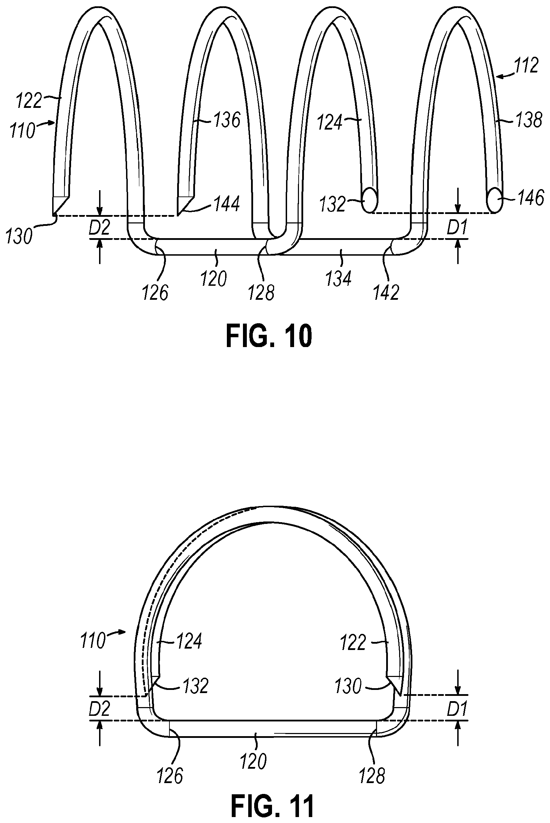

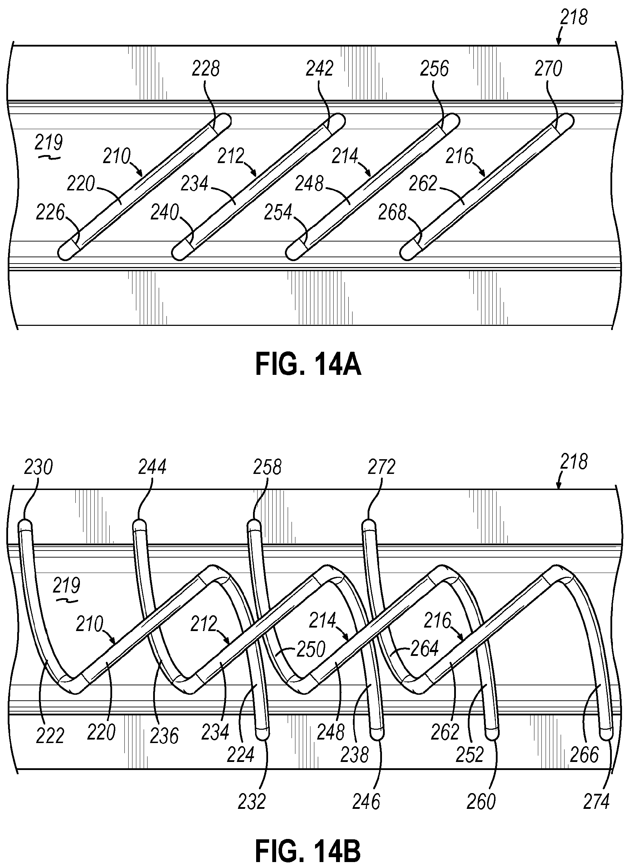

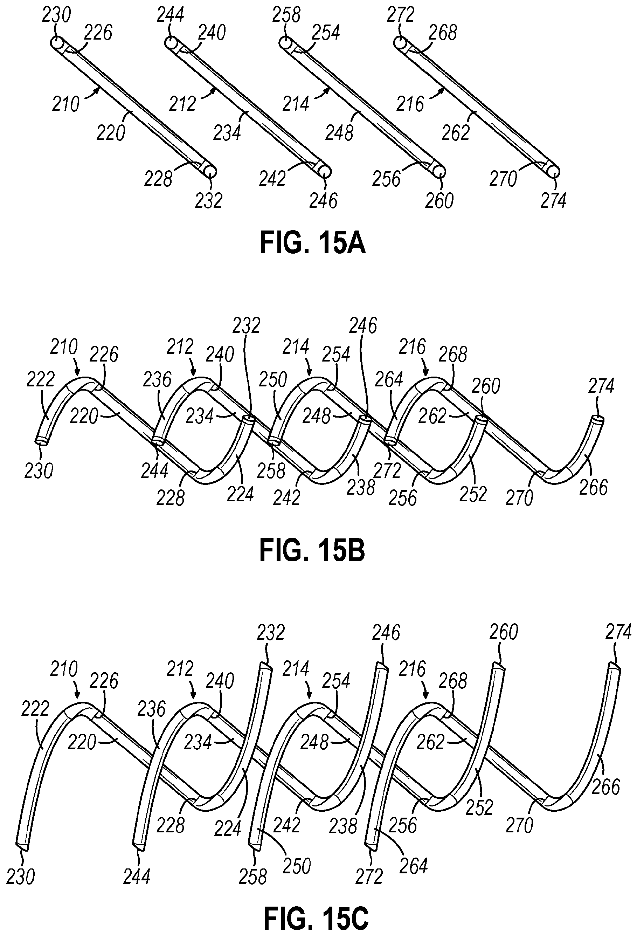

As will be described below with reference to FIGS. 8A-18, exemplary staples (110, 112, 210, 212, 214, 216, 276, 278, 280, 282) may be used instead of staples (47, 310) of staple cartridge (37). Staples (110, 112, 210, 212, 214, 216, 276, 278, 280, 282) may be incorporated into a stapling assembly, which may include features similar to staple cartridge (37) shown and described above with reference to FIGS. 3-7. Portions (e.g., legs) of staples (110, 112, 210, 212, 214, 216, 276, 278, 280, 282, 710) may overlap adjacent staples to form an overlapping staple pattern to increase robustness and strength of the staple line as a whole and which may allow for increased staple density. Staples (110, 112, 210, 212, 214, 216, 276, 278, 280, 282) may be used in conjunction with first and second exemplary alternative anvils (218, 410, 510), and staples (710) may be used in conjunction with a third exemplary alternative anvil (610). Anvils (218, 410, 510, 610) may require reduced time and associated costs of manufacturing, inspecting, and qualifying anvils (218, 410, 510, 610).

### A. First Exemplary Alternative Staples

FIGS. 8-11 show schematic views of first exemplary alternative staples, shown as first and second staples (110, 112), which may be incorporated into the instrument (10) of FIG. 1. While first and second staples (110, 112) are shown, the principles pertaining to first and second staples (110, 112) also apply to additional staples (e.g., a third staple, a fourth staple, a fifth staple, and a sixth staple, etc.).

First staple (110) may exit a first staple aperture (114) of a deck surface (116), which may be generally similar to staple aperture (51) of upper deck (72) shown in FIG. 3. Similarly, second staple (112) may exit a second staple aperture (118) of deck surface (116) similar to first staple aperture (114). First and second staples (110, 112) are configured to transition from a non-deformed state shown in FIGS. 8A and 9A to a deformed state shown in FIGS. 8B and 9B-11 using an anvil (not shown but which may be similar to anvil (18)) when end effector (12) is in the closed configuration of FIG. 4A. First staple (110) is separate and discrete from second staple (112). First staple (110) includes a first crown (120) and a plurality of legs, shown as first and second legs (122, 124). First leg (122) extends from a first end (126) of first crown (120). Similarly, second leg (124) extends from a second end (128) of first crown (120) that is disposed opposite first end (126). First leg (122) includes a first terminal end (130), and second leg (124) includes a second terminal end (132). First and second terminal ends (130, 132) are shown as being sharp and pointed. As shown, first and second legs (122, 124) are generally straight in the non-deformed state. While first and second angles (β1, β2) of first and second legs (122, 124) relative to first crown (120) are shown as being generally equal, first and second angles (β1, β2) may be different.

Similar to first staple (110), second staple (112) includes a second crown (134) and a plurality of legs, which are shown as first and second legs (136, 138). First leg (136) extends from a first end (140) of second crown (134). Similarly, second leg (138) extends from a second end (142) of second crown (134) that is opposite first end (140). First leg (136) includes a first terminal end (144), and second leg (138) includes a second terminal end (146). First and second terminal ends (144, 146) are shown as being sharp and pointed. As shown, first and second legs (136, 138) are generally straight in the non-deformed state.

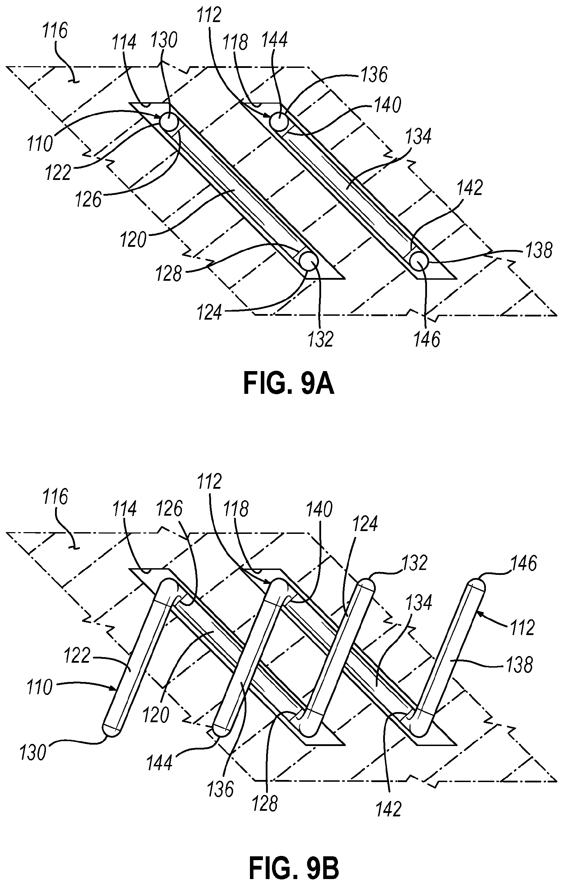

When first staple (110) is transitioned from the non-deformed state of FIGS. 8A and 9A to the deformed state of FIGS. 8B and 9B-11, second leg (124) of first staple (110) is configured to extend over second crown (134) to form an overlapping staple pattern between first and second staples (110, 112). Additionally, when first staple (110) is transitioned from the non-deformed state to the deformed state, first leg (122) of first staple (110) is configured to extend over a crown of another staple (not shown but which may be similar to first and second staples (110, 112)) to form an overlapping staple pattern. Similar to first staple (110), when second staple (112) is transitioned from the non-deformed state to the deformed state, first leg (136) of second staple (112) is configured to extend over first crown (120) of first staple (110) to form an overlapping staple pattern between first and second staples (110, 112). Second leg (138) of second staple (112) is configured to extend over third crown of a third staple (not shown) to form an overlapping staple pattern between second staple (112) and the third staple (not shown) when transitioned to the deformed state. The overlap of first and second staples (110, 112) may increase robustness and strength of the staple line as a whole and may allow for increased staple density.

In the deformed state shown in FIGS. 8B, 10, and 11, first legs (122, 136) and second legs (124, 138) may be bent arcuately. In other words, in the deformed state, first and second legs (122, 124, 136, 138) of first and second staples (110, 112) each form a semicircle that terminates at first and second distances (D1, D2) away from an upper surface of first and second crowns (120, 134). As shown in the deformed state of FIG. 9B, first legs (122, 136) of first and second staples (110, 112) extend generally parallel to one another. Similarly, second legs (124, 138) of first and second staples (110, 112) extend generally parallel to one another and approximately 180 degrees opposite first legs (122, 136). While first and second staples (110, 112) are not shown as being in direct contact with each other, first and second staples (110, 112) appear interleaved together. While first and second distances (D1, D2) are shown as being equal, first and second distances (D1, D2) may be different. Staple drivers (not shown), but which may be similar to staple drivers (43), are configured to contact first and second crowns (120, 134) and drive first and second crowns (120, 134) toward the anvil (e.g., anvil (18)). First and second crowns (120, 134) may extend parallel to one another in each of the non-deformed and deformed states. Each of first and second staples (110, 112) may be configured to contact a staple forming pocket of an anvil (e.g., anvils (218, 410, 510) described below) to transition from the non-deformed state to the deformed state.

### B. Second Exemplary Alternative Staples and First Exemplary Alternative Anvil

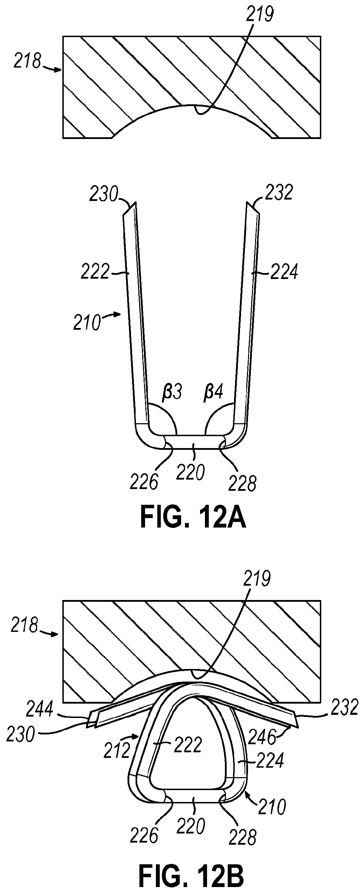

FIGS. 12-18 show schematic views of second exemplary alternative staples, shown as first, second, third, and fourth staples (210, 212, 214, 216), which may be incorporated into the instrument of FIG. 1. While first, second, third, and fourth staples (210, 212, 214, 216) are shown, the principles also apply to additional staples (e.g., a fifth staple, a sixth staple, a seventh staple, an eighth staple, a ninth staple, etc.). First, second, third, and fourth staples (210, 212, 214, 216) are configured to transition from a non-deformed state shown in FIGS. 12A, 13A, 14A, and 15A to a deformed state shown in FIGS. 12B, 13B, 14B, 15C-18 using anvil (218). First, second, third, and fourth staples (210, 212, 214, 216) are separate and discrete from one another.

First staple (210) includes a first crown (220) and a plurality of legs, shown as first and second legs (222, 224). First leg (222) extends from a first end (226) of first crown (220). Similarly, second leg (224) extends from a second end (228) of first crown (220) that is disposed opposite first end (226). First leg (222) includes a first terminal end (230), and second leg (224) includes a second terminal end (232). Similar to first staple (210), second staple (212) includes a second crown (234) and a plurality of legs, which are shown as first and second legs (236, 238). First leg (236) extends from a first end (240) of second crown (234). Similarly, second leg (238) extends from a second end (242) of second crown (234) that is opposite first end (240). First leg (236) includes a first terminal end (244), and second leg (238) includes a second terminal end (246). Similar to first and second staples (210, 212), third staple (214) includes a third crown (248) and a plurality of legs, which are shown as first and second legs (250, 252). First leg (250) extends from a first end (254) of third crown (248). Similarly, second leg (252) extends from a second end (256) of third crown (248) that is opposite first end (254). First leg (250) includes a first terminal end (258), and second leg (252) includes a second terminal end (260).

Similar to first, second, and third staples (210, 212, 214), fourth staple (216) includes a fourth crown (262) and a plurality of legs, which are shown as first and second legs (264, 266). First leg (264) extends from a first end (268) of fourth crown (262). Similarly, second leg (266) extends from a second end (270) of fourth crown (262) that is opposite first end (268). First leg (250) includes a first terminal end (272), and second leg (266) includes a second terminal end (274). First and second terminal ends (230, 232, 244, 246, 258, 260, 272, 274) are shown as being sharp and pointed. As shown in the non-deformed state, first and second legs (222, 224, 236, 238, 250, 252, 264, 266) are generally straight. In the non-deformed state, first leg (236) of second staple (212) is spaced a distance from first crown (220), While first angle (β3) of first leg (222, 236, 250, 264) is shown as being generally equal to second angle (β4) of second leg (224, 238, 252, 266), first and second angles (β3, β4) may be different. Each of first, second, third, and fourth staples (210, 212, 214, 216) may be configured to contact a staple forming pocket (219) of anvil (218) to transition from the non-deformed state to the deformed state. Unlike anvil (18) where each staple (47) has a corresponding staple forming pocket (53), second, third, and fourth staples (210, 212, 214, 216), anvil (218) may be configured to contact the same staple forming pocket (219).

When first staple (210) is transitioned from the non-deformed state to the deformed state, second leg (224) of first staple (210) is configured to extend over second crown (234) to form an overlapping staple pattern between first and second staples (210, 212). Additionally, when first staple (210) is transitioned from the non-deformed state to the deformed state, first leg (222) of first staple (210) is configured to extend over a crown of another staple (not shown), but which may be similar to first, second, third, and fourth staples (210, 212, 214, 216) to form an overlapping staple pattern. Similar to first staple (210), when second staple (212) is transitioned from the non-deformed state to the deformed state, first leg (236) of second staple (212) is configured to extend over first crown (220) of first staple (210) to form an overlapping staple pattern between first and second staples (210, 212). Second leg (238) of second staple (212) is configured to extend over third crown (248) of third staple (214) to form an overlapping staple pattern between second and third staples (212, 214) when transitioned to the deformed state. In the deformed state, first leg (236) is configured to occupy a space between first crown (220) and anvil (218).

Similar to second staple (212), when third staple (214) is transitioned from the non-deformed state to the deformed state, first leg (250) of third staple (214) is configured to extend over second crown (234) of second staple (212) to form an overlapping staple pattern between second and third staples (212, 214). Second leg (252) of third staple (212) is configured to extend over fourth crown (262) of fourth staple (216) to form an overlapping staple pattern between third and fourth staples (214, 216) when transitioned to the deformed state. Similar to third staple (214), when fourth staple (216) is transitioned from the non-deformed state to the deformed state, first leg (264) of fourth staple (216) is configured to extend over third crown (248) of third staple (214) to form an overlapping staple pattern between third and fourth staples (214, 216).

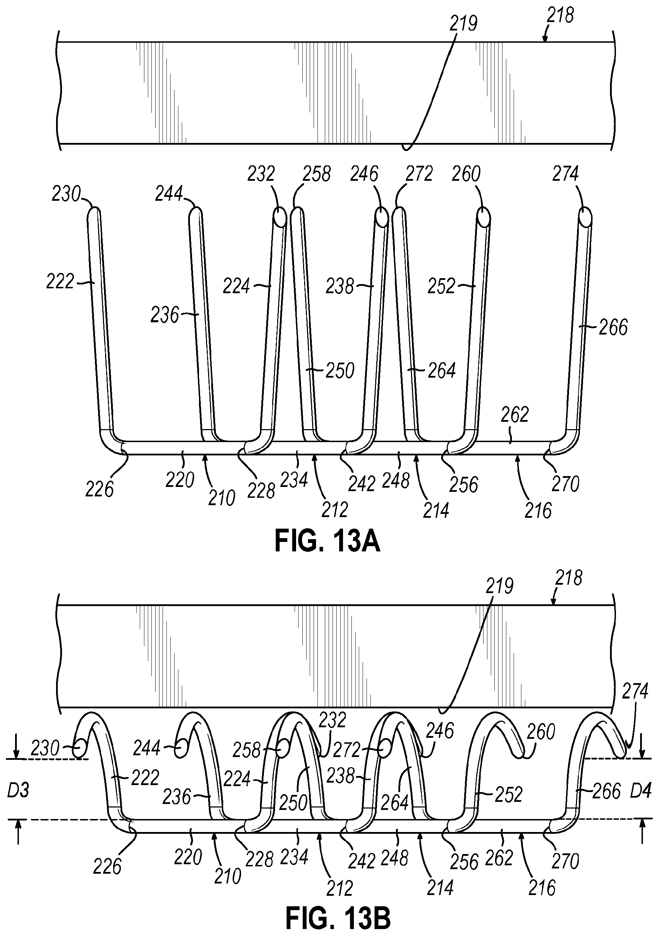

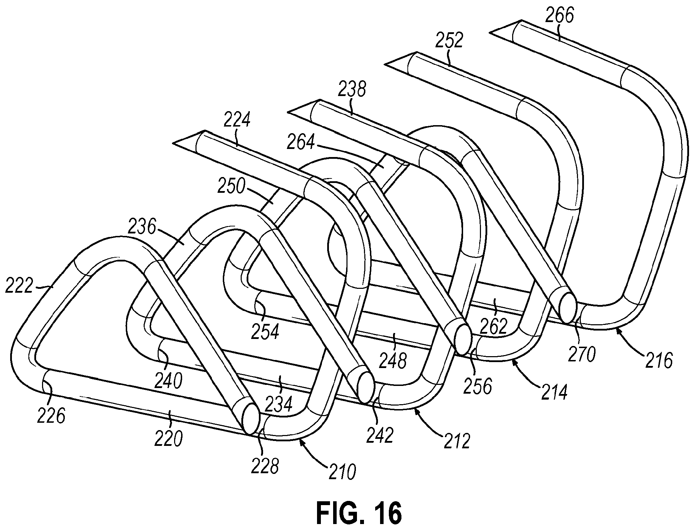

In the deformed state shown in FIGS. 12B, 13B, 14B, 15C-18, first leg (222, 236, 250, 264) and second leg (224, 238, 252, 266) may be bent. In other words, in the deformed state, first and second legs (222, 224, 236, 238, 250, 252, 264, 266) terminate at third and fourth distances (D3, D4) away from an upper surface of first and second crowns (220, 234). While third and fourth distances (D3, D4) are shown in FIG. 13B as being equal, third and fourth distances (D3, D4) may be different. As shown in the deformed state of FIGS. 12B, 13B, 14B, 15C-18, first legs (222, 236, 250, 264) extend generally parallel to one another. Similarly, second leg (224, 238, 252, 266) of first, second, third, and fourth staples (210, 212, 214, 216) extend generally parallel to one another and approximately 180 degrees opposite first legs (222, 236). As shown in FIGS. 12B and 16, first and second legs (222, 224, 236, 238, 250, 252, 264, 266) each include first and second bent portions that extend at an angle relative to each other. While first, second, third, and fourth staples (210, 212, 214, 216) are not shown as being in direct contact with each other, first, second, third, and fourth staples (210, 212, 214, 216) appear interleaved together. Staple drivers (not shown), but which may be similar to staple drivers (43), are configured to contact first, second, third, and fourth crowns (220, 234, 248, 262) may and drive first, second, third, and fourth crowns (220, 234, 248, 262) may toward the anvil (218). First, second, third, and fourth crowns (220, 234, 248, 262) may extend parallel to one another in each of the non-deformed and deformed states.

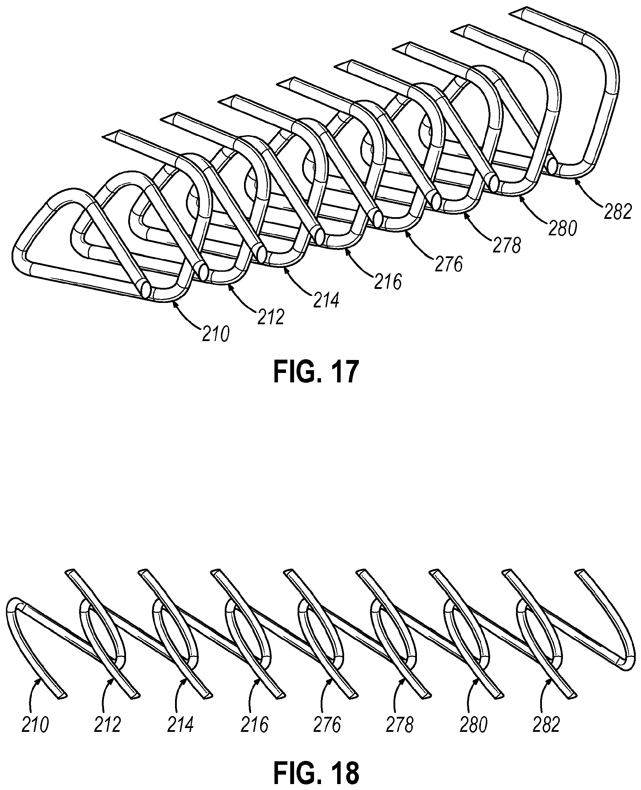

FIGS. 17 and 18 show first, second, third, and fourth staples (210, 212, 214, 216) of FIGS. 12A-16 as well as fifth, sixth, seventh, and eighth staples (276, 278, 280, 282). This illustrates this overlapping pattern may continue with additional staples where legs of adjacent staples are disposed over crowns. The overlap of first, second, third, and fourth, fifth, sixth, seventh, and eighth staples (210, 212, 214, 216, 276, 278, 280, 282) may increase robustness and strength of the staple line as a whole (formed by first, second, third, and fourth staples (210, 212, 214, 216) and may allow for increased staple density.

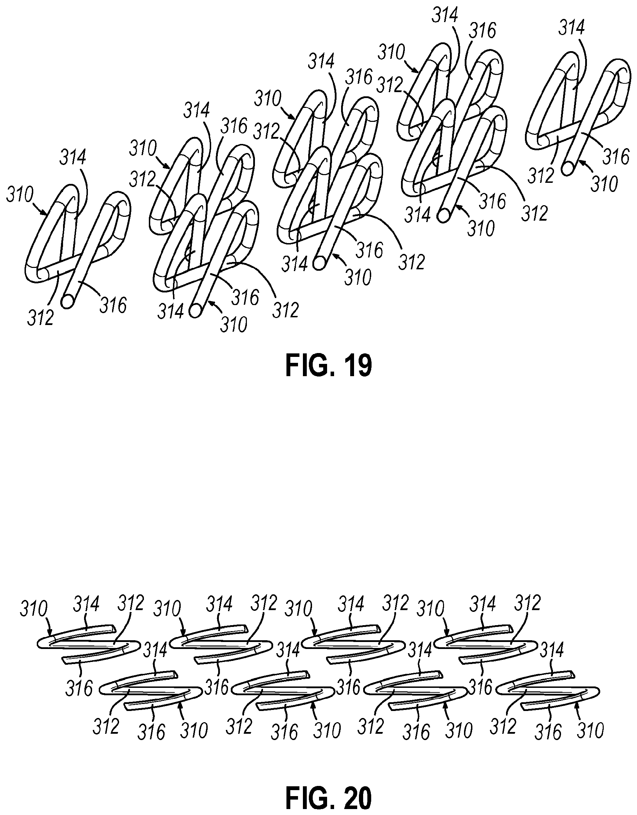

FIGS. 19 and 20 show perspective and top views of exemplary staples (310) that are different than first, second, third, and fourth staples (210, 212, 214, 216) described above. Staples (310) each include a crown (312) with first and second legs (314, 316) extending from crown (312). As shown in FIG. 20, in the deformed state, staples (310) are separated from each another and generally form an S shape.

### C. Second Exemplary Alternative Anvil

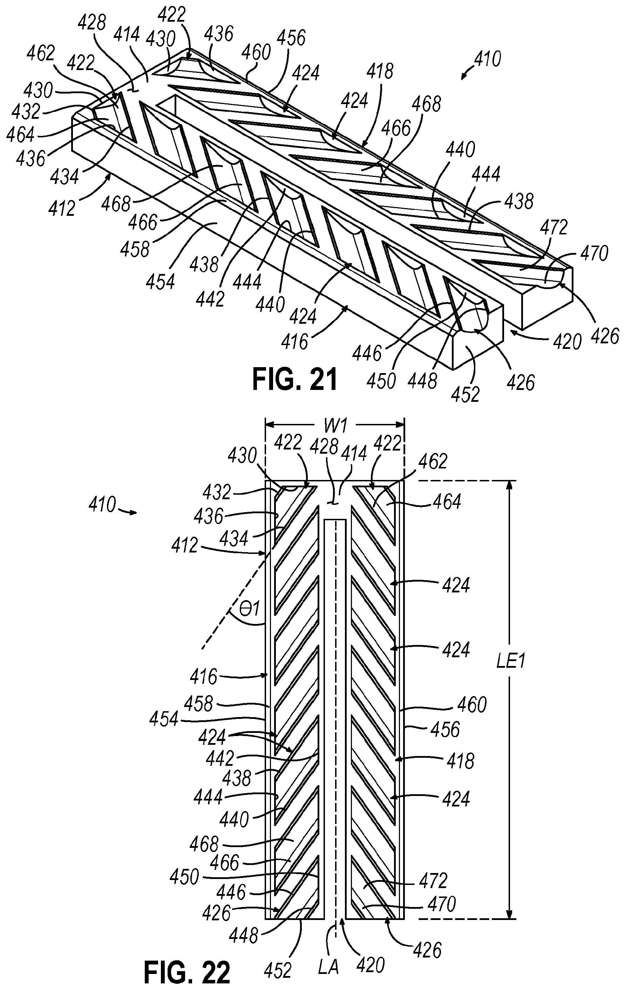

FIGS. 21-22 show a second exemplary alternative anvil (410) that may be incorporated into the instrument (10) of FIG. 1. Anvil (410) includes a body (412), which is shown as being integrally formed together as a unitary piece. Body (412) includes a distal connecting portion (414), a first lateral portion (416), and a second lateral portion (418). First and second lateral portions (416, 418) are separated by a longitudinal slot (420) that is configured to receive a knife (not shown), but which may be similar to distally presented cutting edge (48) of firing beam (14) described above. Anvil (410) is shown as being symmetric about longitudinal slot (420).

Staple forming pockets (422, 424, 426) are disposed at an angle (Θ1) relative to longitudinal axis (LA). Angle (Θ1) may vary. Staple forming pockets (422, 424, 426) slightly angled relative to axis of longitudinal slot (420). Anvil (410) includes distal, central, and proximal staple forming pockets (422, 424, 426) formed in an upper surface (428) of body (412). Distal staple forming pockets (422) include a distal wall (430), first and second angled walls (432, 434), and an outer wall (436). Central staple forming pockets (424) include first and second angled walls (438, 440) and inner and outer walls (442, 444). Proximal staple forming pockets (426) include first and second angled walls (446, 448), an inner wall (450), and open into a proximal end (452) of body (412). First and second angled walls (432, 434, 438, 440, 446, 448) of distal, central, and proximal staple forming pockets (422, 424, 426) extend at angle (Θ1) relative to longitudinal axis (LA). Inner walls (442, 450) and outer wall (444) extend parallel to longitudinal axis (LA). First and second outer lateral walls (454, 456) include tapered outer edges (458, 460). Distal staple forming pockets (422) include a higher slope region (462) and a lower slope region (464). Similarly, central staple forming pockets (424) include a higher slope region (466) and a lower slope region (468) and proximal staple forming pockets (426) include a higher slope region (470) and a lower slope region (472). Higher slope regions (462, 466, 470) have a slope that is greater than lower slope regions (464, 468, 472).

For anvil (410), multiple staples (e.g., staples (110, 112, 210, 212, 214, 216, 276, 278, 280, 282)) may be formed in each staple forming pockets (422, 424, 426). As a result, there may not a 1:1 correlation between staple forming pockets (422, 424, 426) and staples (110, 112, 210, 212, 214, 216, 276, 278, 280, 282). In other words, the number of staples (110, 112, 210, 212, 214, 216, 276, 278, 280, 282) may exceed the number of staple forming pockets (422, 424, 426). Anvil (410) may reduce cost of construction, as staple forming pockets (422, 424, 426) including staple forming pockets (422, 424, 426) that include continuous channels are more inexpensive to manufacture was well as to inspect and qualify. Anvil (410) may allow for increased staple leg density along length (LE1) of anvil (410). Higher staple density may provide increased seal strength against luminal leakage. Anvil (410) may decrease the width (W1) of anvil (410) compared to other layouts of staple forming pockets. Anvil (410) may also reduce the risk associated tipping of staples (110, 112, 210, 212, 214, 216, 276, 278, 280, 282) when moving to the deformed state while still allowing for crossover of adjacent legs (122, 124, 136, 138, 222, 224, 236, 238, 250, 252, 264, 266) relative to crowns (120, 134, 220, 234, 248, 262).

### D. Third Exemplary Alternative Anvil

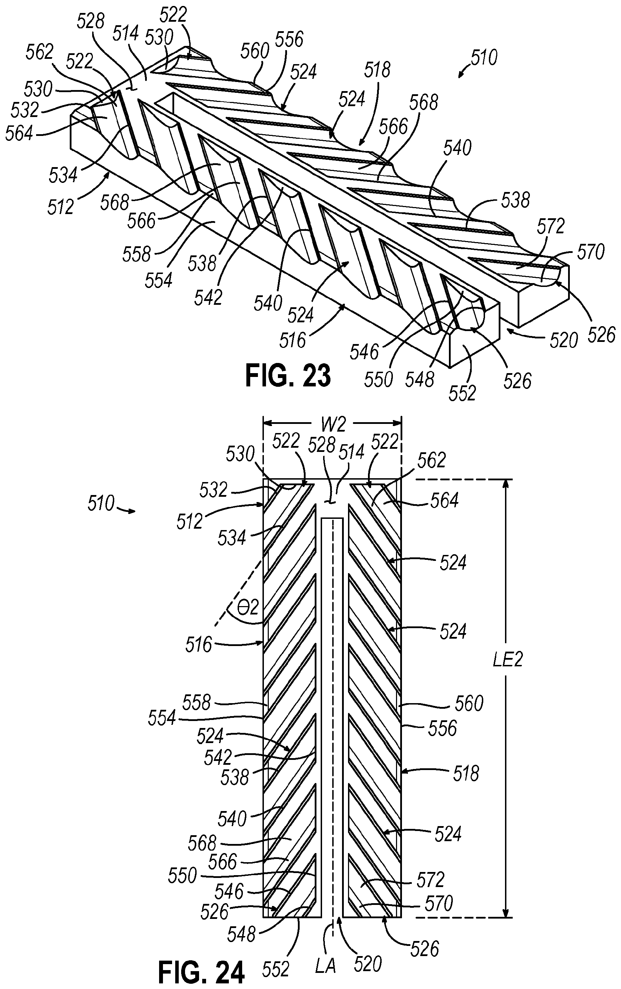

FIGS. 23-24 show a third exemplary alternative anvil (510) that may be incorporated into the instrument (10) of FIG. 1. Anvil (510) is similar to anvil (410) described above. Anvil (510) includes a body (512). Body (512) includes a distal connecting portion (514), a first lateral portion (516), and a second lateral portion (518). First and second lateral portions (516, 518) are separated by a longitudinal slot (520). Anvil (510) is shown as being symmetric about longitudinal slot (520).

Staple forming pockets (522, 524, 526) are disposed at an angle (Θ2) relative to longitudinal axis (LA). Angle (Θ2) may vary. Staple forming pockets (522, 524, 526) slightly angled relative to axis of longitudinal slot (520). Anvil (510) includes distal, central, and proximal staple forming pockets (522, 524, 526) formed in an upper surface (528) of body (512). Distal staple forming pockets (522) include a distal wall (530) and first and second angled walls (532, 534). Central staple forming pockets (524) include first and second angled walls (538, 540) and inner walls (542). Proximal staple forming pockets (526) include first and second angled walls (546, 548), an inner wall (550), and open into a proximal end (552) of body (512). First and second outer lateral walls (554, 556) include tapered outer edges (558, 560). Unlike outer walls (436, 444) of distal and central staple forming pockets (422, 424) described above with reference to FIGS. 20-21, staple forming pockets (522, 524) open directly into first and second outer lateral walls (554, 556) and do not include outer walls (436, 444) of distal and central staple forming pockets (422, 424).

First and second angled walls (532, 534, 538, 540, 546, 548) of distal, central, and proximal staple forming pockets (522, 524, 526) extend at angle (Θ2) relative to longitudinal axis (LA). Inner walls (542, 550) and outer wall (544) extend parallel to longitudinal axis (LA). Distal staple forming pockets (522) include a higher slope region (562) and a lower slope region (564). Similarly, central staple forming pockets (524) include a higher slope region (566) and a lower slope region (568) and proximal staple forming pockets (526) include a higher slope region (570) and a lower slope region (572). For anvil (510), multiple staples (e.g., staples (110, 112, 210, 212, 214, 216, 276, 278, 280, 282)) may be formed in each staple forming pockets (522, 524, 526). As a result, there may not a 1:1 correlation between staple forming pockets (522, 524, 526) and staples (110, 112, 210, 212, 214, 216, 276, 278, 280, 282). In other words, the number of staples (110, 112, 210, 212, 214, 216, 276, 278, 280, 282) may exceed the number of staple forming pockets (522, 524, 526). Anvil (510) may reduce cost of construction as well as be more easily inspected and qualified. Anvil (510) may allow for increased staple leg density along length (LE2) of anvil (510). Higher staple density may provide increased seal strength against luminal leakage. Anvil (510) may decrease the width (W2) of anvil (510) compared to other layouts of staple forming pockets. Anvil (510) may also reduce the risk associated tipping of staples (110, 112, 210, 212, 214, 216, 276, 278, 280, 282) when moving to the deformed state while still allowing for crossover of adjacent legs (122, 124, 136, 138, 222, 224, 236, 238, 250, 252, 264, 266) relative to crowns (120, 134, 220, 234, 248, 262).

### E. Exemplary Circular Surgical Stapler with Fourth Exemplary Alternative Anvil

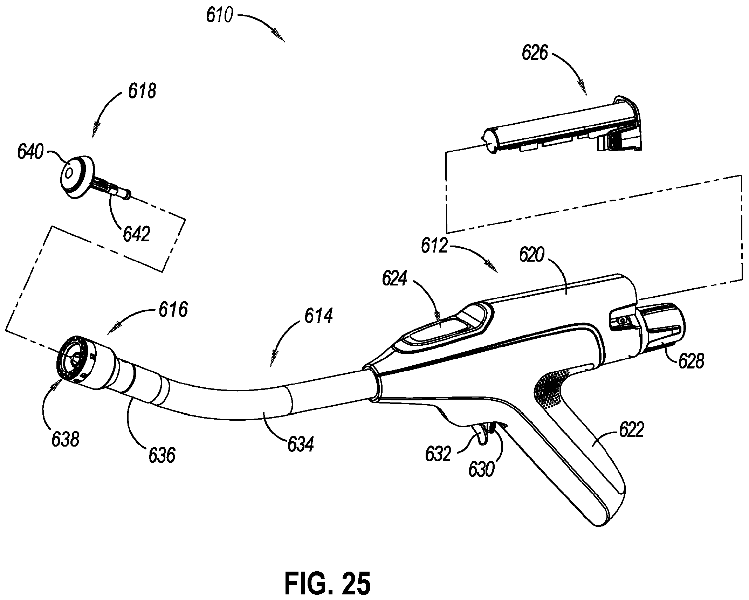

FIG. 25 depicts an exemplary circular surgical stapler (610) that may be used to provide an end-to-end, side-to-side, or end-to-side anastomosis between two sections of an anatomical lumen such as a portion of a patient's digestive tract. Circular surgical stapler (610) of this example comprises a handle assembly (612), a shaft assembly (614), a stapling head assembly (616), and an anvil assembly (618). Handle assembly (612) comprises a casing (620) defining an obliquely oriented pistol grip (622). In some versions, pistol grip (622) is perpendicularly oriented. In some other versions, pistol grip (622) is omitted. Handle assembly (612) further includes a user feedback feature (624) that permits viewing of a movable indicator needle (not shown). Circular surgical stapler (610) includes a battery pack (626). Battery pack (626) is operable to provide electrical power to a motor (not shown). Shaft assembly (614) extends distally from handle assembly (612) and includes a preformed bend, which may facilitate positioning of stapling head assembly (616) within a patient's colon.

Stapling head assembly (616) is located at the distal end of shaft assembly (614). As shown, anvil assembly (618) is configured to removably couple with shaft assembly (614), adjacent to stapling head assembly (616). Anvil assembly (618) and stapling head assembly (616) are configured to cooperate to manipulate tissue in three ways, including clamping the tissue, cutting the tissue, and stapling the tissue. A knob (628) at the proximal end of handle assembly (612) is rotatable relative to casing (620) to provide precise clamping of the tissue between anvil (618) and stapling head assembly (616). When a safety trigger (630) of handle assembly (612) is pivoted away from a firing trigger (632) of handle assembly (612), firing trigger (632) may be actuated to provide cutting and stapling of the tissue. Stapling head assembly (616) of the present example is coupled to a distal end of shaft assembly (614) and comprises a body member (634) and a slidable staple driver member (not shown). Body member (634) includes a distally extending cylindraceous inner core member (not shown). Body member (634) is fixedly secured to an outer sheath (636) of shaft assembly (200). A deck member (638) is fixedly secured to body member (634), and includes a distally presented deck surface (not shown) defining two concentric annular arrays of staple openings. Anvil assembly (618) includes a head (640) and a shank (642).

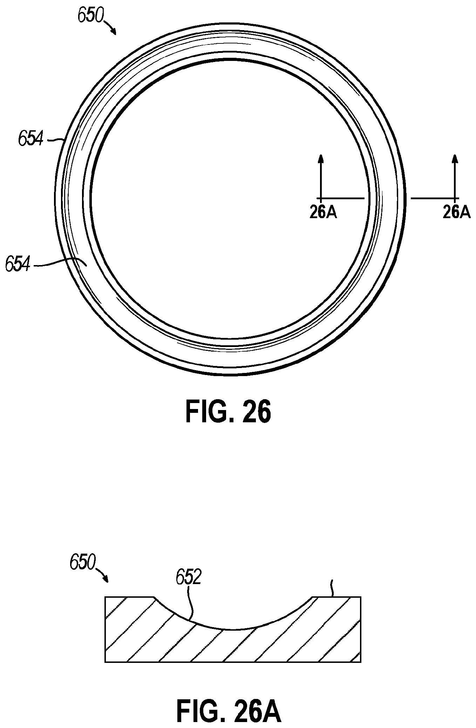

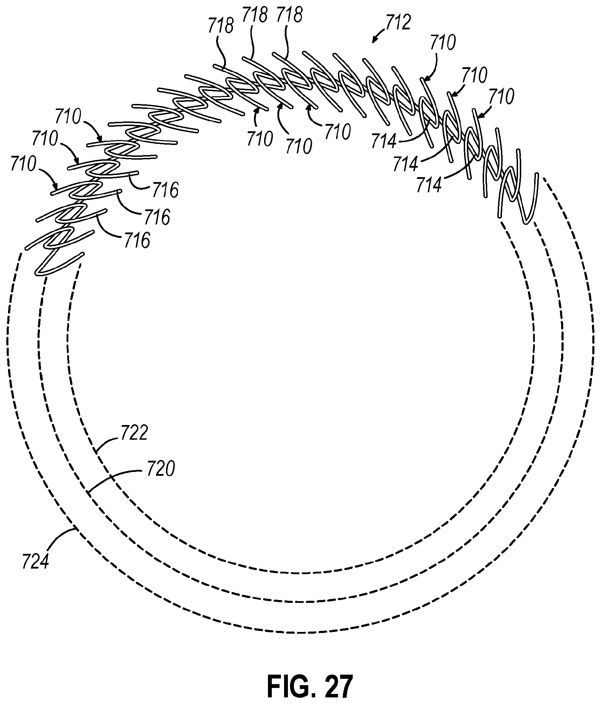

FIGS. 26-26A show a fourth exemplary alternative anvil (650) that may be incorporated into head (640) of anvil assembly (618) of circular surgical stapler (610) shown in FIG. 25. Anvil (650) may be used in conjunction with third exemplary alternative staples (710) of FIG. 27 described below. The principles of anvil (650) and staples (710) may be provided in accordance with one or more teachings of U.S. App. No. 17/401,391, entitled "Methods of Forming an Anastomosis Between Organs with an Expandable Staple Pattern," filed August 13, 2021. The principles of anvil (650) and staples (710) may be provided in accordance with one or more teachings of U.S. Pub. No. 2018/0132849, entitled "Staple Forming Pocket Configurations for Circular Surgical Stapler Anvil," published May 17, 2018; U.S. Pub. No. 2020/0038017, entitled "Surgical End Effectors with Staple Cartridges," published February 6, 2020; and/or U.S. Pat. No. 10,709,452, entitled "Methods and Systems for Performing Circular Stapling," issued July 14, 2020. .

Anvil (650) is shown as being annular. Anvil (650) includes at least one annular staple forming pocket (652) formed in an upper surface (654) of anvil (650). While only one annular staple forming pocket (652) is shown, additional staple forming pockets are also envisioned, which may or may not be annular. For example, annular staple forming pocket (652) may be combined with another annular staple forming pocket (652) and/or another non-annular staple forming pocket. While annular staple forming pocket (652) is shown as being U-shaped in sectional view of FIG. 26A, annular staple forming pocket (652) may have a variety of suitable shapes. Similar to anvils (410, 510), annular staple forming pocket (652) is configured to interact with multiple staples (e.g., staples (110, 112, 210, 212, 214, 216, 276, 278, 280, 282)). In other words, for anvil (650), multiple staples (e.g., staples (110, 112, 210, 212, 214, 216, 276, 278, 280, 282)) may be formed using annular staple forming pocket (652). Anvil (650) may reduce cost of construction, as annular staple forming pocket (652) is more cost effective to produce, to inspect, and to qualify than other staple forming pockets having a 1:1 correlation with respective staples. Anvil (650) may allow for increased staple leg density. Higher staple density may provide increased seal strength against luminal leakage.

### F. Third Exemplary Alternative Staples

FIG. 27 shows third exemplary alternative staples (710) in a deformed state, where the staples (710) may be incorporated into stapling head assembly (616) of circular surgical stapler (610) of FIG. 25. Staples (710) may be similar to staples (110, 112, 210, 212, 214, 216, 276, 278, 280, 282) described above with reference to FIGS. 8A-18. Adjacent staples (710) overlap in a similar manner to staples (110, 112, 210, 212, 214, 216, 276, 278, 280, 282) described above in detail. However, unlike staples (110, 112, 210, 212, 214, 216, 276, 278, 280, 282), staples (710) are arranged in an annular arrangement (712), also referred to as an annular array, for use with stapling head assembly (616) of circular surgical stapler (610). Staples (710) each include a crown (714) with first and second legs (716, 718) extending from crown (714) similar to staples (110, 112, 210, 212, 214, 216, 276, 278, 280, 282). Staples (710) may be loaded into a stapling assembly in a manner allowing for first and second legs (716, 718) to be oriented obliquely to cross over crowns (714) of adjacent staples (710). Staples (710) may be arranged to define a center line (720). In the deformed state, first legs (716) of staples (710) may define an innermost annular point (722) and second legs (718) may define an outermost annular point (724).

### IV. Miscellaneous

Any one or more of the teachings, expressions, embodiments, examples, etc. described herein may be combined with any one or more of the teachings, expressions, embodiments, examples, etc. described in U.S. Pat. App. No. [Atty. Ref. END9370USNP1], entitled "Staple and Staple-Forming Pocket Arrangements for Surgical Staplers," filed on even date herewith.

The teachings of this application may be applied to anvils of all types of surgical staplers, including endocutters, linear surgical staplers, circular surgical staplers, right angle surgical staplers, and curved surgical staplers, for example. For example, the teachings of this application may be combined with various exemplary linear surgical staplers, such that those shown and described in U.S. Pat. No. 11,045,193, entitled "Anvil Assembly for Linear Surgical Stapler," issued June 29, 2021. The teachings of this application may be combined with various exemplary circular surgical staplers, such that those shown and described in U.S. Pat. No. 10,709,452, entitled "Methods and Systems for Performing Circular Stapling," issued July 14, 2020. The teachings of this application may be combined with various exemplary right angle surgical staplers, such that those shown and described in U.S. Pub. No. 2020/0337698, entitled "Tissue Cutting Washer for Right Angle Surgical Stapler," published October 29, 2020. The teachings of this application may be combined with various exemplary curved surgical staplers, such that those shown and described in U.S. App. No. 16/945,042, entitled "Features to Enhance Staple Height Consistency in Curved Surgical Stapler," filed July 31, 2020.

Versions described above may be designed to be disposed of after a single use, or they can be designed to be used multiple times. Versions may, in either or both cases, be reconditioned for reuse after at least one use. Reconditioning may include any combination of the steps of disassembly of the systems, instruments, and/or portions thereof, followed by cleaning or replacement of particular pieces, and subsequent reassembly. In particular, some versions of the systems, instruments, and/or portions thereof may be disassembled, and any number of the particular pieces or parts of the systems, instruments, and/or portions thereof may be selectively replaced or removed in any combination. Upon cleaning and/or replacement of particular parts, some versions of the systems, instruments, and/or portions thereof may be reassembled for subsequent use either at a reconditioning facility, or by an operator immediately prior to a procedure. Those skilled in the art will appreciate that reconditioning of systems, instruments, and/or portions thereof may utilize a variety of techniques for disassembly, cleaning/replacement, and reassembly. Use of such techniques, and the resulting reconditioned systems, instruments, and/or portions thereof, are all within the scope of the present application.

By way of example only, versions described herein may be sterilized before and/or after a procedure. In one sterilization technique, the systems, instruments, and/or portions thereof is placed in a closed and sealed container, such as a plastic or TYVEK bag. The container and system, instrument, and/or portion thereof may then be placed in a field of radiation that can penetrate the container, such as gamma radiation, x-rays, or high-energy electrons. The radiation may kill bacteria on the system, instrument, and/or portion thereof and in the container. The sterilized systems, instruments, and/or portions thereof may then be stored in the sterile container for later use. Systems, instruments, and/or portions thereof may also be sterilized using any other technique known in the art, including but not limited to beta or gamma radiation, ethylene oxide, or steam.

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A surgical stapling instrument, comprising:
(a) an anvil (218) that includes a staple forming pocket (219); and
(b) a stapling assembly comprising:
(i) a first staple (210) comprising:
(A) a first crown (220),
(B) a first leg (222) extending from the first crown, and
(C) a second leg (224) extending from the first crown, and
(ii) a second staple (212), the second staple (212) comprising:
(A) a second crown (234), and
(B) a first leg (236) extending at a first angle relative to
the second crown in a non-deformed state,
wherein the first leg of the second staple (236) is configured to extend over the first crown (220) to form an overlapping staple pattern between the first and second staples when the first and second staples (210, 212) are in a deformed state,
**characterized in that** the staple pocket (219) of the anvil (218) is configured to deform the first and second staples (210, 212) from the non-deformed state to the deformed state.

2. The surgical stapling instrument of claim 1, wherein the second leg of the first staple is disposed at an angle relative to the first crown in the non-deformed state, wherein the second leg of the first staple (224) is configured to extend over the second crown to form an overlapping staple pattern between the first and second staples when the first and second staples are in the deformed state.

3. The surgical stapling instrument of claim 1, wherein the stapling assembly further comprises a third staple (214), wherein the anvil is configured to deform the third staple from a non-deformed state to a deformed state, the third staple comprising:
(A) a third crown (248), and
(B) a first leg (250) disposed at a first angle relative to the third crown in the non-deformed state, wherein the first leg of the third staple is configured to extend over the second crown to form an overlapping staple pattern between the second and third staples when the second and third staples are in the deformed state.

4. The surgical stapling instrument of claim 3, wherein the second staple further comprises a second leg (238) disposed at a second angle relative to the second crown in the non-deformed state, wherein the second leg (238) is configured to extend over the third crown to form an overlapping staple pattern between the second and third staples when the second and third staples are in the deformed state.

5. The surgical stapling instrument of claim 4, wherein the first and second angles of the first and second legs of the second staple (236, 238) are equal.

6. The surgical stapling instrument of claim 4, wherein the first and second legs of the second staple (236, 238) are straight in the non-deformed state.

7. The surgical stapling instrument of claim 4, wherein the stapling assembly further comprises a fourth staple (216), wherein the anvil is configured to deform the fourth staple to thereby transition the fourth staple (216) from a non-deformed state to a deformed state, the fourth staple comprising:
(A) a fourth crown (262), and
(B) a first leg (264) disposed at a first angle relative to the fourth crown in the non-deformed state, wherein the first leg of the fourth staple (262) is configured to extend over the third crown of the third staple to form an overlapping staple pattern between the third and fourth staples when the third and fourth staples are in the deformed state.

8. The surgical stapling instrument of claim 1, wherein the stapling assembly extends along in a longitudinal direction, wherein the first leg of the second staple (236) is spaced a distance from the longitudinal direction from the first crown in the non-deformed state, wherein in the deformed state the first leg of the second staple (236) is configured to occupy a space between the first crown and the anvil.

9. The surgical stapling instrument of claim **1,** wherein the first and second crowns (220, 234) are configured to extend parallel to each another in the non-deformed and deformed states.

10. The surgical stapling instrument of claim **1,** wherein the first staple (210) is discrete from the second staple (212) in the non-deformed and deformed states.

11. The surgical stapling instrument of claim 1, further comprising a first staple driver configured to contact the first crown and drive the first crown toward the anvil.

12. The surgical stapling instrument of claim **1,** wherein the stapling assembly extends along a longitudinal axis, wherein the staple forming pocket (219) is disposed at an oblique angle relative to the longitudinal axis of the stapling assembly.

13. The surgical stapling instrument of claim **1,** further comprising an annular array of staples that include the first and second staples.

14. The surgical stapling instrument of claim 13, wherein the anvil includes an annular anvil having a single annular staple forming pocket to interact with the annular array of staples.

15. The surgical stapling instrument of claim **1,** wherein the second leg of the first staple (224) is configured to extend over the second crown (234) to form an overlapping staple pattern between the first and second staples (210, 212) when the first and second staples are in the deformed state.

## Patentansprüche

1. Chirurgisches Klammerinstrument, umfassend:
(a) einen Amboss (218), der eine Klammerformungstasche (219) einschließt; und
(b) eine Klammeranordnung, umfassend:
(i) eine erste Klammer (210), umfassend:
(A) eine erste Krone (220),
(B) einen ersten Schenkel (222), der sich von der ersten Krone aus erstreckt, und
(C) einen zweiten Schenkel (224), der sich von der ersten Krone aus erstreckt, und
(ii) eine zweite Klammer (212), die zweite Klammer (212) umfassend:
(A) eine zweite Krone (234) und
(B) einen ersten Schenkel (236), der sich in einem ersten Winkel relativ zu der zweiten Krone in einem nicht verformten Zustand erstreckt,
wobei der erste Schenkel der zweiten Klammer (236) konfiguriert ist, um sich über die erste Krone (220) zu erstrecken, um ein überlappendes Klammermuster zwischen der ersten und der zweiten Klammer zu formen, wenn sich die erste und die zweite Klammer (210, 212) in einem verformten Zustand befinden,
**dadurch gekennzeichnet, dass** die Klammertasche (219) des Ambosses (218) konfiguriert ist, um die erste und die zweite Klammer (210, 212) aus dem nicht verformten Zustand in den verformten Zustand zu verformen.

2. Chirurgisches Klammerinstrument nach Anspruch 1, wobei der zweite Schenkel der ersten Klammer in dem nicht verformten Zustand in einem Winkel relativ zu der ersten Krone eingerichtet ist, wobei der zweite Schenkel der ersten Klammer (224) konfiguriert ist, um sich über die zweite Krone zu erstrecken, um ein überlappendes Klammermuster zwischen der ersten und der zweiten Klammer zu formen, wenn sich die erste und die zweite Klammer in dem verformten Zustand befinden.

3. Chirurgisches Klammerinstrument nach Anspruch 1, wobei die Klammeranordnung ferner eine dritte Klammer (214) umfasst, wobei der Amboss konfiguriert ist, um die dritte Klammer von einem nicht verformten Zustand in einen verformten Zustand zu verformen, die dritte Klammer umfassend:
(A) eine dritte Krone (248) und
(B) einen ersten Schenkel (250), der in einem ersten Winkel relativ zu der dritten Krone in dem nicht verformten Zustand eingerichtet ist, wobei der erste Schenkel der dritten Klammer konfiguriert ist, um sich über die zweite Krone zu erstrecken, um ein überlappendes Klammermuster zwischen der zweiten und der dritten Klammer zu formen, wenn sich die zweite und die dritte Klammer in dem verformten Zustand befinden.

4. Chirurgisches Klammerinstrument nach Anspruch 3, wobei die zweite Klammer ferner einen zweiten Schenkel (238) umfasst, der in dem nicht verformten Zustand in einem zweiten Winkel relativ zu der zweiten Krone eingerichtet ist, wobei der zweite Schenkel (238) konfiguriert ist, um sich über die dritte Krone zu erstrecken, um ein überlappendes Klammermuster zwischen der zweiten und der dritten Klammer zu formen, wenn sich die zweite und die dritte Klammer in dem verformten Zustand befinden.

5. Chirurgisches Klammerinstrument nach Anspruch 4, wobei der erste und der zweite Winkel des ersten und des zweiten Schenkels der zweiten Klammer (236, 238) gleich sind.

6. Chirurgisches Klammerinstrument nach Anspruch 4, wobei der erste und der zweite Schenkel der zweiten Klammer (236, 238) in dem nicht verformten Zustand gerade sind.

7. Chirurgisches Klammerinstrument nach Anspruch 4, wobei die Klammeranordnung ferner eine vierte Klammer (216) umfasst, wobei der Amboss konfiguriert ist, um die vierte Klammer zu verformen, um dadurch die vierte Klammer (216) von einem nicht verformten Zustand in einen verformten Zustand zu überführen, die vierte Klammer umfassend:
(A) eine vierte Krone (262) und
(B) einen ersten Schenkel (264), der in einem ersten Winkel relativ zu der vierten Krone in dem nicht verformten Zustand eingerichtet ist, wobei der erste Schenkel der vierten Klammer (262) konfiguriert ist, um sich über die dritte Krone der dritten Klammer zu erstrecken, um ein überlappendes Klammermuster zwischen der dritten und der vierten Klammer zu formen, wenn sich die dritte und die vierte Klammer in dem verformten Zustand befinden.

8. Chirurgisches Klammerinstrument nach Anspruch 1, wobei sich die Klammeranordnung in einer Längsrichtung erstreckt, wobei der erste Schenkel der zweiten Klammer (236) in dem nicht verformten Zustand einen Abstand von der Längsrichtung von der ersten Krone entfernt ist, wobei in dem verformten Zustand der erste Schenkel der zweiten Klammer (236) konfiguriert ist, um einen Raum zwischen der ersten Krone und dem Amboss einzunehmen.

9. Chirurgisches Klammerinstrument nach Anspruch 1, wobei die erste und die zweite Krone (220, 234) konfiguriert sind, um sich in dem nicht verformten und dem verformten Zustand parallel zueinander zu erstrecken.

10. Chirurgisches Klammerinstrument nach Anspruch 1, wobei die erste Klammer (210) in dem nicht verformten und dem verformten Zustand von der zweiten Klammer (212) getrennt ist.

11. Chirurgisches Klammerinstrument nach Anspruch 1, ferner umfassend einen ersten Klammertreiber, der konfiguriert ist, um mit der ersten Krone in Kontakt zu kommen und die erste Krone in Richtung des Ambosses zu treiben.

12. Chirurgisches Klammerinstrument nach Anspruch 1, wobei sich die Klammeranordnung entlang einer Längsachse erstreckt und die Klammerformungstasche (219) in einem schrägen Winkel relativ zu der Längsachse der Klammeranordnung eingerichtet ist.

13. Chirurgisches Klammerinstrument nach Anspruch 1, ferner umfassend eine ringförmige Gruppierung von Klammern, die die erste und die zweite Klammer einschließen.

14. Chirurgisches Klammerinstrument nach Anspruch 13, wobei der Amboss einen kreisförmigen Amboss, der eine einzelne kreisförmige Klammerformungstasche aufweist, die mit der kreisförmigen Gruppierung von Klammern interagiert, einschließt.

15. Chirurgische Klammerinstrument nach Anspruch 1, wobei der zweite Schenkel der ersten Klammer (224) konfiguriert ist, um sich über die zweite Krone (234) zu erstrecken, um ein überlappendes Klammermuster zwischen der ersten und der zweiten Klammer (210, 212) zu formen, wenn sich die erste und die zweite Klammer in dem verformten Zustand befinden.

## Revendications

1. Instrument d'agrafage chirurgical, comprenant :
(a) une enclume (218) qui comporte une poche de formation d'agrafe (219) ; et
(b) un ensemble d'agrafage comprenant :
(i) une première agrafe (210) comprenant :
(A) une première barre transversale (220),
(B) une première branche (222) s'étendant à partir de la première barre transversale, et
(C) une seconde branche (224) s'étendant à partir de la première barre transversale, et
(ii) une deuxième agrafe (212), la deuxième agrafe (212) comprenant :
(A) une deuxième barre transversale (234), et
(B) une première branche (236) s'étendant selon un premier angle par rapport à la deuxième barre transversale dans un état non déformé,
dans lequel la première branche de la deuxième agrafe (236) est conçue pour s'étendre par-dessus la première barre transversale (220) pour former un motif d'agrafes en chevauchement entre les première et deuxième agrafes lorsque les première et deuxième agrafes (210, 212) sont dans un état déformé,
**caractérisé en ce que** la poche d'agrafe (219) de l'enclume (218) est conçue pour déformer les première et deuxième agrafes (210, 212) de l'état non déformé à l'état déformé.

2. Instrument d'agrafage chirurgical selon la revendication 1, dans lequel la seconde branche de la première agrafe est disposée selon un angle par rapport à la première barre transversale dans l'état non déformé, dans lequel la seconde branche de la première agrafe (224) est conçue pour s'étendre par-dessus la deuxième barre transversale pour former un motif d'agrafes en chevauchement entre les première et deuxième agrafes lorsque les première et deuxième agrafes sont dans l'état déformé.

3. Instrument d'agrafage chirurgical selon la revendication 1, dans lequel l'ensemble d'agrafage comprend en outre une troisième agrafe (214), dans lequel l'enclume est conçue pour déformer la troisième agrafe d'un état non déformé à un état déformé, la troisième agrafe comprenant :
(A) une deuxième barre transversale (248), et
(B) une première branche (250) disposée selon un premier angle par rapport à la troisième barre transversale dans l'état non déformé, dans lequel la première branche de la troisième agrafe est conçue pour s'étendre par-dessus la deuxième barre transversale pour former un motif d'agrafes en chevauchement entre les deuxième et troisième agrafes lorsque les deuxième et troisième agrafes sont dans l'état déformé.

4. Instrument d'agrafage chirurgical selon la revendication 3, dans lequel la deuxième agrafe comprend en outre une seconde branche (238) disposée selon un second angle par rapport à la deuxième barre transversale dans l'état non déformé, dans lequel la seconde branche (238) est conçue pour s'étendre par-dessus la troisième barre transversale pour former un motif d'agrafes en chevauchement entre les deuxième et troisième agrafes lorsque les deuxième et troisième agrafes sont dans l'état déformé.

5. Instrument d'agrafage chirurgical selon la revendication 4, dans lequel les premier et second angles des première et seconde branches de la deuxième agrafe (236, 238) sont égaux.

6. Instrument d'agrafage chirurgical selon la revendication 4, dans lequel les première et seconde branches de la deuxième agrafe (236, 238) sont linéaires dans l'état non déformé.

7. Instrument d'agrafage chirurgical selon la revendication 4, dans lequel l'ensemble d'agrafage comprend en outre une quatrième agrafe (216), dans lequel l'enclume est conçue pour déformer la quatrième agrafe pour faire passer de ce fait la quatrième agrafe (216) d'un état non déformé à un état déformé, la quatrième agrafe comprenant :
(A) une quatrième barre transversale (262), et
(B) une première branche (264) disposée selon un premier angle par rapport à la quatrième barre transversale dans l'état non déformé, dans lequel la première branche de la quatrième agrafe (262) est conçue pour s'étendre par-dessus la troisième barre transversale de la troisième agrafe pour former un motif d'agrafes en chevauchement entre les troisième et quatrième agrafes lorsque les troisième et quatrième agrafes sont dans l'état déformé.

8. Instrument d'agrafage chirurgical selon la revendication 1, dans lequel l'ensemble d'agrafage s'étend dans une direction longitudinale, dans lequel la première branche de la deuxième agrafe (236) est espacée à une distance de la direction longitudinale par rapport à la première barre transversale dans l'état non déformé, dans lequel dans l'état déformé la première branche de la deuxième agrafe (236) est conçue pour occuper un espace entre la première barre transversale et l'enclume.

9. Instrument d'agrafage chirurgical selon la revendication 1, dans lequel les première et deuxième barres transversales (220, 234) sont conçues pour s'étendre parallèles l'une à l'autre dans les états non déformé et déformé.

10. Instrument d'agrafage chirurgical selon la revendication 1, dans lequel la première agrafe (210) est distincte de la deuxième agrafe (212) dans les états non déformé et déformé.

11. Instrument d'agrafage chirurgical selon la revendication 1, comprenant en outre un premier dispositif d'entraînement d'agrafes conçu pour venir en contact avec la première barre transversale et entraîner la première barre transversale en direction de l'enclume.

12. Instrument d'agrafage chirurgical selon la revendication 1, dans lequel l'ensemble d'agrafage s'étend le long d'un axe longitudinal, dans lequel la poche de formation d'agrafe (219) est disposée selon un angle oblique par rapport à l'axe longitudinal de l'ensemble d'agrafage.

13. Instrument d'agrafage chirurgical selon la revendication 1, comprenant en outre un réseau annulaire d'agrafes qui comportent les première et deuxième agrafes.

14. Instrument d'agrafage chirurgical selon la revendication 13, dans lequel l'enclume comporte une enclume annulaire ayant une unique poche de formation d'agrafe annulaire pour interagir avec le réseau annulaire d'agrafes.

15. Instrument d'agrafage chirurgical selon la revendication 1, dans lequel la seconde branche de la première agrafe (224) est conçue pour s'étendre par-dessus la deuxième barre transversale (234) pour former un motif d'agrafes en chevauchement entre les première et deuxième agrafes (210, 212) lorsque les première et deuxième agrafes sont dans l'état déformé.
